Europäisches Patentamt

European Patent Office

Office européen des brevets

(11) Publication number: **0 434 149 A2**

# EUROPEAN PATENT APPLICATION

(21) Application number: 90203333.1

(22) Date of filing: **13.12.90**

(51) Int. Cl.⁵: **B01L 11/00, G01N 1/00**

(30) Priority: **22.12.89 US 455548**

(43) Date of publication of application:
**26.06.91 Bulletin 91/26**

(84) Designated Contracting States:
**BE CH DE FR GB IT LI LU NL**

(71) Applicant: **EASTMAN KODAK COMPANY**
**343 State Street**
**Rochester, NY 14650-0221(US)**

(72) Inventor: **Columbus, Richard Lewis, c/o**
**EASTMAN KODAK COMPANY**
**Patent Department, 343 State Street**
**Rochester, New York 14650-2201(US)**

(74) Representative: **Phillips, Margaret Dawn et al**
**Kodak Limited Patent Department Headstone**
**Drive**
**Harrow, Middlesex HA1 4TY(GB)**

(54) **Liquid transfer apparatus.**

(57) It is well known to dispense quantities of liquid on to test elements using pipettes. However, due to peculiar properties of many body liquids, great care is needed to ensure that accurate and reproducible amounts of such liquids are dispensed. Disclosed herein is dispensing apparatus which can be used in combination with standard pipettes to allow accurate predetermined amounts of liquid to be dispensed. The dispensing apparatus includes a liquid transfer unit (20) which comprises a frame (22) having a liquid inlet aperture (30) connected by a first passageway (34, 35) to a dispensing aperture (28), an air vent aperture (42) connected by a second passageway (44) which extends toward the first passageway (34, 35), and a valve (50, 52, 54, 56) interposed between the two passageways (34, 35, 44). The liquid aperture (30) is shaped to receive a number of conventional pipettes. The valve (50, 52, 54, 56) allows continuous liquid flow along the first passageway (34, 35) when in a first position, or continuous air flow along the second passageway (44) and into part of the first passageway (35) when in a second position to effect dispensing of the liquid.

FIG. 3

## LIQUID TRANSFER APPARATUS

This invention relates to liquid transfer apparatus and in particular, apparatus which allows any pipette to be used without careful control of manual positions, to accurately dispense small amounts of liquids for clinical analysis.

The dispensing of aliquots of a body liquid such as blood serum onto a dried, slide-like test element is a key first step in the analysis of the liquid in an analyzer. Because of the peculiar properties of many body liquids, and the need for accurate and reproducible dispensing of a predetermined aliquot, great care has been taken in prior art devices to ensure that a) the proper tip is used by the dispensing apparatus to direct the flow properly, and/or b) the dispensing tip is properly presented and positioned at the test element at the time of dispensing. Examples of the technology which achieve feature a) above include tips of the type described in US-A-4347875. Examples of the technology used to achieve features b) above include dispensing means in analyzers of the type described in US-A-4340390; US-A-4452899 and US-A-4615360. In the latter three, the analyzer is constructed to carefully prepare the dispensing tip just prior to dispensing of the aliquot, for example, in its spacing from the test element and/or by blowing off any exterior liquid hanging on the outside of the tip.

Such care as expressed by such technologies has worked well. However, they all require expensive, peculiar apparatus which is costly either in its construction or its use. Although this is not particularly disadvantageous when constructing an expensive, high-volume analyzer, it is a drawback when constructing an inexpensive, low-volume analyzer, such as might be needed or used in remote field locations.

It is therefore an object of the present invention to provide an inexpensive interface which will allow a conventional pipette to dispense liquid which still flows on to the test element in a predictable, accurate manner.

More specifically, in accordance with one aspect of the present invention, there is provided liquid transfer apparatus for transferring a predetermined amount of liquid from a pipette on to a test element, the apparatus comprising:-
an inlet aperture;
a dispensing aperture for dispensing some of the liquid in droplet form;
a first liquid flow path extending from the inlet aperture towards the dispensing aperture;
an air vent;
a second liquid flow path extending from the vent towards the first flow path; and

valve means is interposed between the first and second liquid flow paths for allowing continuous liquid flow either from the inlet aperture to the dispensing aperture, or from the vent to the dispensing aperture with the inlet aperture blocked; characterized in that the apparatus further includes a support for supporting a test element, the test element having an upper surface which is to receive the dispensed liquid, and spacer means for accurately positioning the apparatus from the support at a predetermined distance such that an initial droplet from the dispensing aperture contacts the upper surface of the test element while still in contact with the dispensing aperture.

In accordance with another aspect of the present invention, there is provided a method of dispensing accurately a predetermined amount of liquid on to a test element, comprising the steps of:-

a) injecting more than the predetermined amount of liquid from a pipette into liquid transfer apparatus as described above;
b) moving the valve means to block flow between the inlet aperture and the dispensing aperture and to fluidly connect the liquid adjacent the dispensing aperture with the air vent; and
c) applying an amount of air pressure to the air vent which is effective to accurately dispense the predetermined amount of liquid out of the dispensing aperture.

It is an advantageous feature of the invention that any pipette can be used manually, and without careful control, to dispense accurate aliquots of a body liquid in the proper way onto a test element for assaying.

It is a related advantageous feature that an inexpensive interface is provided which receives any pipette positioned without great care, and dispenses liquid received from the pipette in the proper way.

The present invention will now be described by way of example only, with reference to the accompanying drawings in which:-

Figure 1 is a fragmentary perspective view of a first embodiment of liquid transfer apparatus constructed in accordance with the present invention;

Figure 2 is a vertical sectioned view taken generally along the line II-II of Figure 1;

Figure 3 is a sectioned view similar to that shown in Figure 2, but illustrating the parts as they are positioned for the dispensing step;

Figure 4 is a plan view of another embodiment of liquid transfer apparatus constructed in accordance with the present invention; and

Figures 5A and 5B are fragmentary sectioned views of the apparatus shown in Figure 4, showing valve means in the alternative liquid filling position and liquid dispensing position respectively.

The invention is hereinafter described with respect to the preferred embodiments, which feature the use of particular kinds of valves to control the two flow paths to dispense preferably blood serum or a reference liquid. Additionally, the invention is useful regardless of the type of liquid being dispensed and with apparatus using other kinds of valves, so long as the valve is effective to allow either the one flow path or the other flow path to be effective.

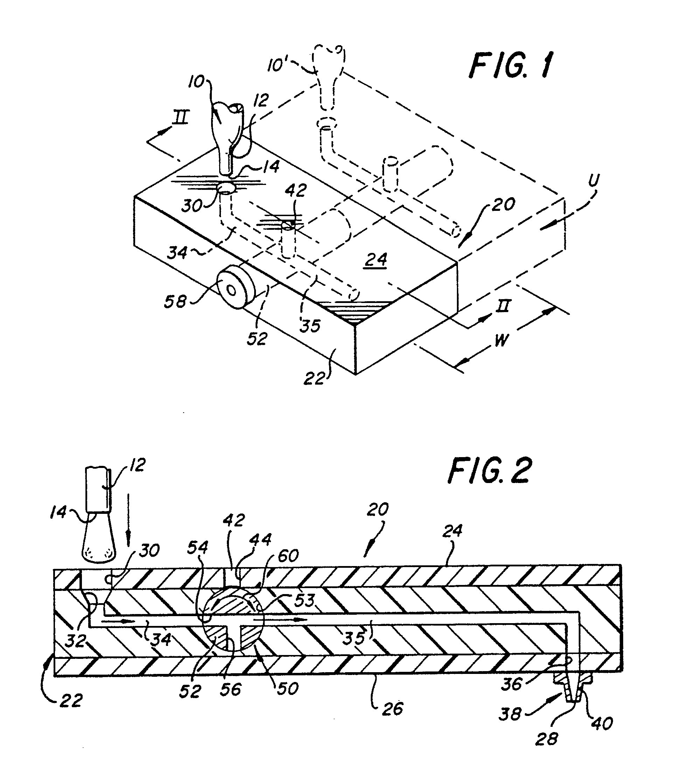

As shown in Figures 1 to 3, apparatus in accordance with the present invention features a pipette 10 and liquid transfer apparatus 20. The pipette 10 can be any pipette whatsoever, of any construction, conventional or otherwise, having a tip portion 12 with an outlet 14. Tip portion 12 can be fixed to or removable from the rest of the pipette 10. It is because of the interface provided by apparatus 20 that it does not matter what pipette is used.

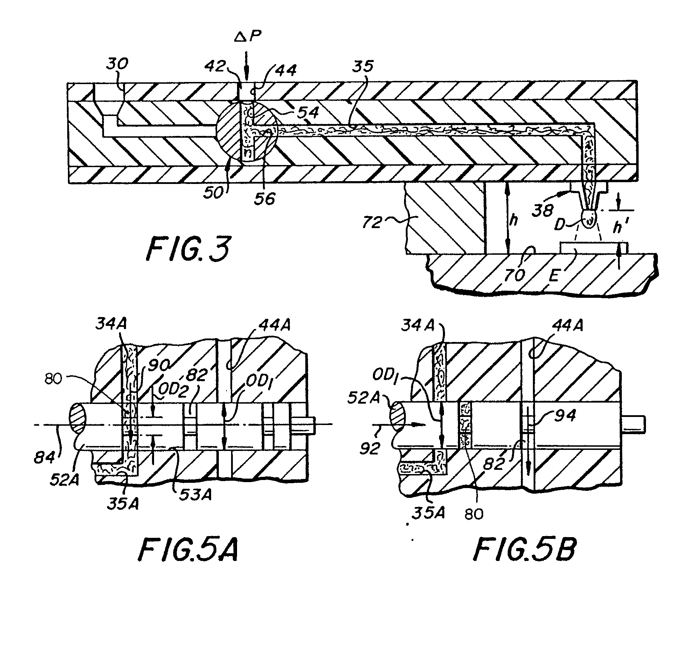

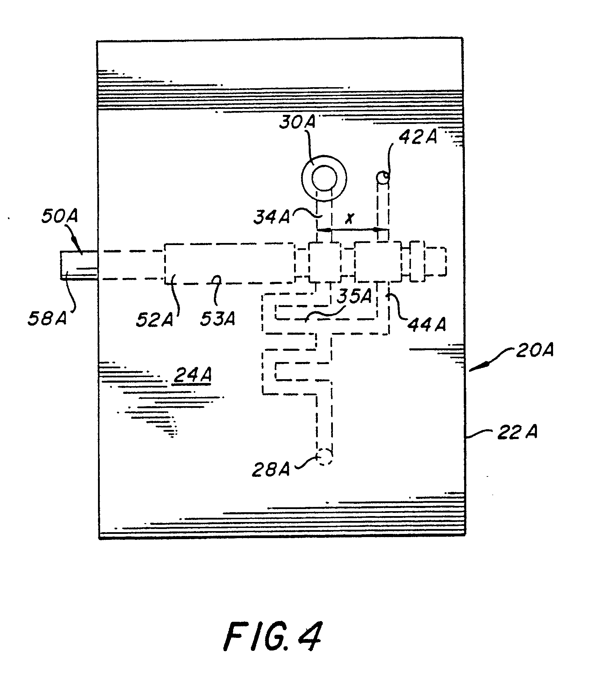

Apparatus 20 comprises a frame 22, which can be any shape, a rectangular slab being shown by way of example. Top surface 24 is constructed to engage the pipette and a source of $\Delta P$ air pressure (not shown). A bottom surface 26, shown more clearly in Figure 2, is provided to supply a dispensing aperture 28 and a proper spacing from a test element E, as shown in Figure 3, and will be described in more detail hereinafter.

More specifically, top surface 24 has an inlet aperture 30, shaped to receive tip portion 12 directly, or to receive liquid ejected therefrom. Preferably, the pipette 10 is actually inserted, to seat on a surface 32 at the bottom of aperture 30, as shown in Figure 2.

Aperture 30 fluidly connects with a fixed passageway 34, 35 which extends to an orifice 36 in surface 26. Valve 50 described hereinafter is disposed partway along passageway 34, 35. An appropriate dispensing tip 38 is mounted at orifice 36 to fluidly connect its aperture 28 with orifice 36.

Any kind of tip 38 can be used, provided it is shaped to discourage perfusion up outside surface 40 thereof, and instead directs flow, such as a drop D (shown in Figure 3) on to the element E. A useful example (not shown) is a tip configured as described in US-A-4347875.

A vent aperture 42 is provided, such as in top surface 24 (Figure 2) and fluidly connects with a passageway 44 which extends towards passageway 34. Preferably, passageway 44 has a width sufficiently large as to discourage capillary attraction of liquid out of passageway 56.

Between the two passageways, valve 50 is interposed to allow, as alternatives, the completion of passageway 34-35 or the completion of passageway 44-35. A three-way stop cock valve, here shown as cylindrically shaped, is useful. The valve 50 comprises a cylinder 52, disposed in a bore 53, having a diameter passageway 54, a radial passageway 56 extending from, and at an angle of 90° to passageway 54, and a handle means 58 exterior of frame 22 for rotating the valve 50 as shown in Figure 1. Appropriate seals, not shown, are included to prevent axial leakage along the cylinder 52.

In use, valve 50 is rotated to the position as shown in Figure 2. In this position, passageway 34 is connected to passageway 35 via passageway 54. As liquid is ejected from an inserted pipette 10, the liquid fills passageways 34, 35 until it reaches dispensing aperture 28.

Thereafter, cylinder 52 is rotated, in the direction of arrow 60, until passageway 54 is aligned with passageway 44, and passageway 56 is aligned with passageway 35, as shown in Figure 3. In this position, valve 50 has blocked off the inlet aperture 30. The application of pressure $\Delta P$ at aperture 42 will cause the dispensing of an aliquot of the liquid from passageway 35 and tip 38, onto element F. Most preferably, $\Delta P$ is generated from external air pressure delivered via means such as a hose (not shown) connected to aperture 42. (The hose is shaped to seal at aperture 42).

In order to ensure that the proper dispensing height h' is achieved (see Figure 3), surface 26 is positioned the proper height h above the support surface 70 of element E. Any suitable mechanism can be used to obtain such heights. For example, a spacer block 72 can be utilized, which can be a separate element or can be an integral part of apparatus 20.

Alternatively, apparatus 20 can be constructed to provide multiple transfer opportunities for a multiple number of pipettes 10, 10' containing each a different liquid, as is indicated in phantom in Figure 1. In particular, a plural number of passageways 34, 35 can be provided in parallel, each with an inlet aperture 30, to accommodate a ganged pipette 10, 10'. In such a construction, cylinder 52 can extend the full width of the entire device, so that handle means 58 is effective to rotate all the valves simultaneously. Such a construction is useful to allow the dispensing of serum, from pipette 10 and a reference liquid from pipette 10' for example. Particularly for such an example, the dispensing tips, of which only the first one 38 is depicted (Figure 2), preferably would be spaced apart a distance w, as shown in Figure 1, which would allow the simultaneous dispensing of serum and reference liquid onto a single ISE test element of the type described in US-A-4184936, for example.

Alternatively, (not shown), each of the separate passageways 34,35 can be valved separately from the others, in which case cylinder 52 would not extend across the entire unit. Instead, each passageway 34,35 would have its own cylinder and own operating handle means 58.

Still further, an additional option is to provide a temporary seal (not shown) over aperture 30 after liquid is inserted via the pipette, to allow storage in apparatus 20 before the dispensing step.

By reason of this transfer apparatus, the user need not be concerned about whether the pipette 10 has a tip 12 which is particularly suited for accurate dispensing (that is, will discourage perfusion). Nor need the user carefully position the pipette 10 relative to any particular vertical position, since apparatus 20 automatically provides the correct height h' between the dispensing orifice and element E, as shown in Figure 3. As a result, an initial droplet D forms on tip 38 and then contacts element E while still in contact with aperture 28 of tip 38 to provide proper dispensing (shown in phantom in Figure 3).

Tips 38 can be different in length, if plural units U are ganged together, as shown in Figure 1, and if a different height h' is needed at the adjacent apertures. For example, liquid used for radial wash preferably is applied with a height which is less than h' used for serum dispensing.

It is not essential that the valve be a rotating type as shown. Alternatively, for example, it can be a spool valve as shown in Figures 4, 5A and 5B. Parts similar to those previously described bear the same reference numeral, to which the distinguishing suffix A is appended.

Thus, in Figure 4, apparatus 20A comprises a frame 22A providing a top surface 24A in which an inlet aperture 30A and a vent aperture 42A are provided. A passageway 34A, 35A fluidly connects apertures 30A and 42A with a dispensing aperture 28A, via a valve 50A, also as in the previous embodiment. However, in this embodiment, valve 50A is a spool valve comprising a cylinder 52A which slides, rather than rotates, within bore 53A. Outer diameter $OD_1$, as shown in Figure 5A, seals within bore 53A, whereas a reduced inner diameter $OD_2$ is provided at two locations 80, 82 spaced apart along axis 84 of cylinder 52A. The spacing of locations 80 and 82 is constructed to be less than the spacing x (shown in Figure 4) of passageway 34A from passageway 44A which extends from the vent aperture 42A. In this embodiment, passageway 44A does not feed into the extension of passageway 34A, 35A which occurs within the valve, but rather joins passageway 35A downstream from valve 50A. In particular, there is no intersection of the air path and liquid path within the valve, as in the previous embodiment, but rather downstream thereof.

In use, cylinder 52A is slid so that reduced diameter $OD_2$ at location 80 is aligned with passageway 34A,35A as shown in Figure 5A. Liquid is inserted from the pipette (not shown), to provide continuous flow, in the direction of arrow 90, towards the dispensing aperture. In this position, cylinder 52A has $OD_2$ at location 82 which is misaligned with air passageway 44A. Thereafter, valve 50A is moved, using handle 58A (Figure 4) by sliding cylinder 50A sideways, in the direction of arrow 92 as shown in Figure 5B, so that outer diameter $OD_1$ blocks any flow from passageway 34A to 35A, and at the same time $OD_1$ at location 82 becomes aligned with the parts of passageway 44A. This allows air pressure to flow continuously, in the direction of arrow 94, from the air vent to passageway 35A, as shown in Figure 4, and hence, to the liquid therein. When valve 50A is positioned as shown in Figure 5B, air pressure is applied continuously in an amount effective to accurately dispense a predetermined aliquot, for example 10 $\mu l$, of liquid out of the dispensing aperture.

Passageway 35A can be serpentine in its construction, as shown in Figure 4, or it can be more linear in the manner of its construction shown in Figure 2.

## Claims

1. Dispensing apparatus (20; 20A) for transferring a predetermined amount of liquid from a pipette (10; 10A) on to a test element (F), the apparatus comprising:-

   an inlet aperture (30; 30A);

   a dispensing aperture (28; 28A) for dispensing some of the liquid in droplet form;

   a first liquid flow path (34, 35; 34A, 35A) extending from the inlet aperture (30; 30A) towards the dispensing aperture (28; 28A);

   an air vent (42; 42A);

   a second liquid flow path (44; 44A) extending from the vent (42; 42A) towards the first flow path (34, 35; 34A, 35A); and

   valve means (50, 52, 54, 56; 50A, 52A) is interposed between the first and second liquid flow paths (34, 35, 44; 34A, 35A, 44A) for allowing continuous liquid flow either from the inlet aperture (30; 30A) to the dispensing aperture (28; 28A), or from the vent (42; 42A) to the dispensing aperture (28; 28A) with the inlet aperture (30; 30A) blocked;

   characterized in that the apparatus further includes a support (70) for supporting a test element (F), the test element (E) having an upper surface which is to receive the dispensed liquid, and spacer means (72) for accurately positioning the apparatus (20; 20A) from the support (70) at a predetermined distance (h) such that an initial droplet (D) from the dispensing aperture (28; 28A) contacts the upper surface of the test element (E) while still in contact with the dispensing aperture (28; 28A).

2. Apparatus according to claim 1, wherein the valve means (50, 52, 54, 56) is interposed at the junction of the first and second liquid flow paths (34, 35, 44).

3. Apparatus according to claim 2, wherein the valve means comprises a rotating valve (50, 52, 54, 56).

4. Apparatus according to claim 1, wherein the valve means (50A, 52A) intersects the first and second liquid flow paths (34A, 35A, 44A) at two different locations.

5. Apparatus according to claim 4, wherein the valve means comprises a spool valve (50A, 52A).

6. Apparatus according to any one of the preceding claims, further including a pipette (10; 10A).

7. A method of dispensing accurately a predetermined amount of liquid on to a test element, comprising the steps of :-
   a) injecting more than the predetermined amount of liquid from a pipette (10; 10A) into liquid transfer apparatus (20; 20A) according to any one of the preceding claims;
   b) moving the valve means (50, 52, 54, 56; 50A, 52A) to block flow between the inlet aperture (30; 30A) and the dispensing aperture (28; 28A) and to fluidly connect the liquid adjacent the dispensing aperture (28; 28A) with the air vent (42; 42A); and
   c) applying an amount of air pressure ($\Delta P$) to the air vent (42; 42A) which is effective to accurately dispense the predetermined amount of liquid out of the dispensing aperture (28; 28A).

FIG. 1

FIG. 2

FIG.3

FIG.5A

FIG.5B

EP 0 434 149 A2

FIG. 4